# EUROPEAN PATENT SPECIFICATION

(11) **EP 4 262 685 B1**
(45) Date of publication and mention of the grant of the patent: **14.10.2026**
(21) Application number: 21811379.3
(22) Date of filing: 23.11.2021
(51) Int. Cl.: A61Q 1/02, A61Q 19/08, G01J 3/46, A61K 8/19, A61K 8/25, A61K 8/891, A61K 8/29

(54) **METHOD FOR SELECTING PERSONALIZED SKIN CARE COMPOSITION**
VERFAHREN ZUR AUSWAHL EINER PERSONALISIERTEN HAUTPFLEGEZUSAMMENSETZUNG
PROCÉDÉ DE SÉLECTION D'UNE COMPOSITION DE SOINS DE LA PEAU PERSONNALISÉE

(30) Priority: 18.12.2020 WO PCT/CN2020/137593; 27.01.2021 EP 21153652
(43) Date of publication of application: 25.10.2023
(73) Proprietor: Unilever IP Holdings B.V., 6708 WH Wageningen (NL); Unilever Global IP Limited, Wirral, Merseyside CH62 4ZD (GB)
(72) Inventor: FU, Chenyang, Shanghai 200335 (CN); SUN, Lei, Shanghai 200335 (CN); WANG, Xiaoli, Shanghai 200335 (CN)
(74) Representative: Unilever Patent Group
(86) International application number: PCT/EP2021/082567
(87) International publication number: WO 2022/128355

(56) References cited:
- EP-A1- 3 553 732
- EP-A1- 3 563 719
- EP-A2- 0 462 709
- WO-A1-2017/071886
- US-B1- 6 603 550

## Description

### Field of the invention

The present invention relates to a method for selecting a personalized skin care composition for providing at least one cosmetic benefit selected from pore reduction and wrinkle reduction for an individual. Such method is capable of providing a skin care composition particular efficient for that individual.

### Background of the invention

Appearance of an individual changes with age. These changes become visible in various skin attributes such as, wrinkles formation, pores, age spots and etc. These attributes, in particular wrinkles and pores cause imperfection in the appearance of skin, which may detract from the overall appearance of an individual and negatively affect the personal image.

Thus, there have been considerable efforts by the cosmetics industry to provide skin care compositions which can mask or at least attenuate skin imperfections. More and more consumers are seeking skin care composition to keep their pores and/or wrinkles from looking larger than they really are. Currently, it is generally that the consumers themselves to choose a skin care composition to treat their enlarged skin pores, aged wrinkles to visually minimizing the appearance of these imperfections. US Patent application US-B1-6,603,550 discloses a method for selecting colour cosmetics optimized to a customer's skin shade with a specific device. European patent application EP-A 1-3553732 discloses a method for selecting the colour of a cosmetic product to be applied to the skin based on the actual skin tone of a person. European patent application EP-A2-0462709 discloses foundation-type make up compositions containing a powder phase comprising spherical silica, nylon particles having, Iron oxide and titanium dioxid. European patent application EP-A 1-3563719 discloses concentrated pigment drops providing a form of personalized cosmetics whereby the consumers choose pigment drops that best suit their skin tone. International patent application. WO-A1-2017/071886 discloses compositions to maintain a youthful appearance (evenness of skin tone, reduction of wrinkles) comprising turbostratic boron nitride and porous silica, a silicone elastomers powder and whitening pigments.

Interestingly, the present inventors found that a skin care composition capable of significantly visually reducing the appearance of the imperfections of skin of one user, particularly the skin pore and/or wrinkles, may not be that efficient to other users. Therefore, the present inventor developed a method for selecting a personalized skin care composition for providing at least one cosmetic benefit selected from pore reduction and wrinkle reduction for an individual through matching the color of the skin surface in need of treatment with the color of a skin care composition. In such way, the efficiency is significantly improved for that individual.

### Summary of the invention

In a first aspect, the present invention is directed to a method for selecting a personalized skin care composition for providing at least one cosmetic benefit selected from pore reduction and wrinkle reduction for an individual, the method comprising the steps of determining the color of a skin surface in need of treatment of the individual, and choosing a skin care composition having substantially same color as the skin surface in need of treatment, wherein a) the composition comprises a polymer particle and b) substantially same color means that the color difference (Delta E) of the skin surface before and after the application of the skin care composition with a dosage of 2 mg/cm² is 0 to 5.5 in CIELAB color space..

In a second aspect, the present invention is directed to a method for providing at least one cosmetic benefit selected from pore reduction and wrinkle reduction for an individual comprising the step of determining the color of a skin surface in need of treatment of the individual, choosing a skin care composition having substantially same color as the skin surface in need of treatment, and applying the skin care composition to the skin surface in need of treatment of the individual, wherein a) the composition comprises a polymer particle and b) substantially same color means that the color difference (Delta E) of the skin surface before and after the application of the skin care composition with a dosage of 2 mg/cm² is 0 to 5.5 in CIELAB color space..

In a third aspect, the present invention is directed to a system to select a personalized skin care composition for providing at least one cosmetic benefit selected from pore reduction and wrinkle reduction for an individual, comprising a measuring unit to determine the color of a skin surface of the individual, and a recommending unit to choose a skin care composition having substantially same color as the skin surface in need of treatment, wherein a) the composition comprises a polymer particle and b) substantially same color means that the color difference (Delta E) of the skin surface before and after the application of the skin care composition with a dosage of 2 mg/cm² is 0 to 5.5 in CIELAB color space..

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

"Diameter" as used herein refers to particle diameter in non-aggregated state unless otherwise stated. For polydisperse samples having particulate with diameter no greater than 1 µm, diameter means the z-average diameter measured, for example, using dynamic light scattering (see international standard ISO 13321) with an instrument such as a Zetasizer Nano^{™} (Malvern Instruments Ltd, UK) unless otherwise stated. For polydisperse samples having particulate with diameter no less than 1 µm, diameter means the apparent volume median diameter (D50, also known as x50 or sometimes d(0.5)) of the particles measurable for example, by laser diffraction using a system (such as a Mastersizer^{™} 2000 available from Malvern Instruments Ltd) meeting the requirements set out in ISO 13320 unless otherwise stated.

"Specific surface area" as used herein refers to specific surface area determined according to Brunauer-Emmett-Teller method. The value of the specific surface area was measured by meeting the requirements set out in ASTM standard D 3663-78.

To fully understand the preferred embodiment of the present invention, some methods of color measurement will be described. The color may be characterized by three properties, i.e. hue, chroma and lightness. Each color can be described and distinguished from other colors using these three properties. Hue is generally perceived to be the "color" of a color, for example, red, blue and etc. Chroma generally describes the vividness or dullness of a color. Lightness typically refers to the luminous intensity of a color.

Color space can be described as a method for expressing the color of an object using some kind of notation, such as numbers. One typical color space is CIELAB color space (also known as CIE L*a*b* or sometimes informally abbreviated as "Lab"), defined by the International Commission on Illumination (abbreviated CIE) in 1976. In CIELAB color space, L* is the lightness of the color, a* is the position between green (negative values) and red/magenta (positive values), and b* is the position between blue (negative values) and yellow (positive values). Another one is and CIELCH (also known as CIE L*c*h* or sometimes informally abbreviated as "Lch") color space. The CIELCH color space is similar to CIELAB, but it describes color differently using cylindrical coordinates instead of rectangular coordinates. In CIELCH color space, L* indicates lightness, c* represents chroma, and h* is the hue angle.

The step of determining color of a skin surface in need of treatment of the individual may be conducted in any suitable way. However, it is preferable that the step of determining color comprises determining all of hue, chroma and lightness of the skin surface or all values of L*, a*, and b* of the skin surface. More preferably, the step of determining color of a skin surface of the individual comprises determining all values of L*, a*, and b* of the skin surface.

Generally, the step of determining color of a skin surface of the individual may be conducted by human naked eyes, comparing the color of skin surface with a color swatch, and/or use of an appropriate instrument, preferably comparing the color of skin surface with a color swatch, and/or use of an appropriate instrument. More preferably, the step of step of determining color is conducted by a colorimeter and/or by a spectrophotometer.

Preferably, the step of choosing a skin care composition comprises: a) applying a skin care composition onto the skin surface, b) measuring the color of skin surface coated with skin care composition, c) comparing the colors of coated skin surface and uncoated skin surface; and d) taking the skin care composition if the colors of colors of coated skin surface and uncoated skin surface are substantially same, or repeating the steps of a), b) and c) until the color of the coated skin surface and uncoated is substantially same.

Alternatively or additionally, the step of choosing a skin care composition comprises the step of comparing the color of the skin surface in need of treatment with a database and identifying a skin care composition having substantially same color as the skin surface in need of treatment. The database contains information relating to the color of the skin care compositions and the color of the skins, as well as information relating to the appropriate skin care composition wherein before and after the application of the skin care composition the colors of the skin surface are substantially same. Preferably, this database is formed through testing on a wide range of skin surfaces of individuals and/or fake skins, and skin care composition, so as to produce a library in which the skin care composition is matched with skin surface with specific color. Alternatively, or additionally, it will also be appreciated that the database base may be obtained by virtue of mathematical correlations obtained between the color of skin surface and color of the skin care composition.

Alternatively, or additionally, the step of selecting may comprise a graphical representation or printed table which has the color of the skin surface the selected skin care composition.

Substantially same color means that the color of the skin surface before and after application of the skin care composition is substantially indiscernible to the naked human eye, under normal, ambient light conditions. In this regards, substantially same color may be expressed in terms of a CIELAB color space. Substantially same color may be two points on the color space wherein the values for L*, a* and b* for each point are each sufficiently close, for example differing by less than a predetermined Delta E number. Delta E is a single number that represents the amount of difference between two colors, or if testing a single swatch, the amount of change that particular color has undergone. As used in the present invention, substantially same color typically means that the color difference (Delta E) of the skin surface before and after the application of the skin care composition with a dosage of 2 mg/cm² is 0 to 5.5, preferably 0 to 4.5, more preferably 0 to 3.4, and even more preferably 0 to 2.5, in CIELAB color space.

The pore reduction typically refers to visibly reduce the size of skin pores. It also comprises for example, reducing skin pores, minimizing skin pores, and/or shrinking skin pore. The wrinkle reduction typically refers to visibly reduce the appearance of wrinkle, particularly through physical way. Preferably, the cosmetic benefit is pore reduction.

The skin care composition preferably comprises pigment. Pigment is practically insoluble particle, preferably it has a primary particle size of 0.01 to 10 µm, where the distance represents the longest dimension of the primary particle. The primary particle size may be measured by scanning electron microscopy. Most preferably the pigments have a primary particle size between 0.2 to 2 µm. Preferably, the total amount of pigment is 0.00001 to 10%, more preferably from 0.001 to 6%, even more preferably from 0.1 to 3% by weight of the total composition. Preferably, if the composition comprises titanium dioxide, the amount of the titanium dioxide is less than 4%, more preferably less than 3% by weight of the composition.

Preferably, the composition comprises coloured pigment and/or white pigment. Preferably the pigment comprises iron oxide, and titanium dioxide. Coloured pigment as used herein refers to the pigment which is not white. The amount of coloured pigment of is preferably in the range of 0.00001 to 10%, more preferably from 0.001 to 5%, even more preferably from 0.01 to 2% by weight of the total composition.

Suitable coloured pigment may be inorganic or organic, but preferably the coloured pigment comprises inorganic pigment. The coloured pigment includes, but are not limited to, D&C Red No. 36 and D&C Orange No.17, the calcium lakes of D&C Red Nos. 7,11, 31 and 34, the barium lake of D&C Red No.12, the strontium lake D&C Red No.13, the aluminum lakes of FD&C Yellow No. 5, of FD&C Yellow No.6, of D&C Red No.27, of D&C Red No.21, and of FD&C Blue No.1, iron oxides, manganese violet, chromium oxide, ultramarine blue, and carbon black particles.

The coloured pigment particularly preferably comprises iron oxide. These often are mixtures of differently coloured iron oxides which may be red, yellow, brown and black.

In more preferred embodiments, the pigment comprises coloured pigment and white pigment. Preferably, the white pigment comprises titanium dioxide, zinc oxide, barium oxide, talc, calcium carbonate or a mixture thereof. More preferably, the white pigment is selected from titanium dioxide, zinc oxide, barium oxide, or a mixture thereof. Even more preferably, the white pigment comprises titanium dioxide and most preferably the white pigment is titanium dioxide.

The white pigment is preferably present in amount of 0.0001 to 10% by weight of the composition, more preferably from 0.001 to 5%, even more preferably from 0.01 to 3% by weight of the composition.

Preferably, the skin care composition comprises a polymer particle having an average diameter of 0.2 to 80 microns. Preferably the polymer particle has an average diameter of 0.3 to 50 microns, more preferably from 0.5 to 30 microns, even more preferably from 0.8 to 20 microns, and still even more preferably from 1.5 to 15 microns. For sake of clarity, the polymer particle is different from the pigment in the composition. Preferably, the polymer particle is preferably present in amount of 0.5 to 50%, more preferably 3 to 38%, even more preferably from 5 to 33%, still even more preferably from 10 to 28% by weight of the composition.

Preferably, the polymer particle is a copolymer particle, and more preferably a crosslinked copolymer particle. Preferably, the polymer particle is selected from silicone elastomer, or polymer comprises a polyester or acrylic monomer, or combination thereof. More preferably, the polymer particle is selected from a silicone elastomer, a polyester or combination thereof. Even more preferably the polymer particle is selected from dimethicone/vinyl dimethicone crosspolymer, dimethicone crosspolymer, Polysilicone-11, polyester of adipic acid and neopentyl glycol or a mixture thereof.

The composition preferably comprises a silicone elastomer. The silicone elastomer may either be an emulsifying or non-emulsifying cross-linked silicone elastomer or a combination thereof but preferably the silicone elastomer is non-emulsifying. The term "non-emulsifying," as used herein, defines cross-linked silicone elastomer from which poly-oxyalkylene units are absent. The term "emulsifying," as used herein, means crosslinked organo-polysiloxane elastomer having at least one poly-oxyalkylene (e.g., polyoxyethylene or poly-oxypropylene) unit.

Preferred silicone elastomers are organo-polysiloxanes available under the INCI (International Nomenclature of Cosmetic Ingredients) names of dimethicone/vinyl dimethicone crosspolymer, dimethicone crosspolymer and Polysilicone-11. More preferably the silicone elastomer is dimethicone/vinyl dimethicone crosspolymer.

The silicone elastomer is preferably present in amount of 0.5 to 50%, more preferably 3 to 38%, even more preferably from 5 to 33%, still even more preferably from 10 to 28% by weight of the composition.

The composition preferably comprises a polyester. Preferably the polyester is a crosslinked polyester. Preferably, the crosslinked polyester may be formed by reacting a C₄-C₈ diacid with a branched C₄-C₆ diol. More preferably, the polyester is a polyester of adipic acid and neopentyl glycol. Even more preferably the polyester is a polyester of adipic acid and neopentyl glycol crosslinked with isopropyltriethylsilane. Still even more preferably the polyester is the polymer has INCI name of adipic acid/neopentyl glycol crosspolymer.

Preferably, the composition comprises an inorganic particle. Preferably, the inorganic particle is porous. Inorganic particle herein also includes an inorganic particle core with surface modified by organic material. For the avoidance of doubt, the inorganic particle is different from either the pigment or the polymer particle. The inorganic particle preferably has an average diameter of 200 nm to 40 microns, more preferably from 0.6 to 25 microns, even more preferably from 1 to 20 microns, still even more preferably from 1.5 to 12 microns and most preferably from 2 to 5 microns. To have a better sensory, the inorganic particle is preferably substantially uniform in size which means less than 5% of the inorganic particles have a diameter less than 0.5 times the average diameter and less than 5% of the inorganic particles have a diameter greater than 1.5 times the average diameter. In another aspect, the range of the diameter of the inorganic particle is preferably 0.8 to 1.2 times the average diameter, more preferably 0.9 to 1.1 times the average diameter. The inorganic particle is preferably present in amount of 0.01 to 20% by weight of the composition, more preferably from 0.05 to 14%, even more preferably from 0.2 to 9%, still even more preferably from 0.4 to 5% and most preferably from 0.8 to 3% by weight of the composition.

Preferably, the inorganic particle is silica, preferably porous silica. The porous silica is preferably non-fumed silica. Preferably, the porous silica is hydrophilic. Even more preferably the porous silica is unmodified porous silica microsphere. Hydrophilic porous silica as used herein refers to silica having a water absorption value of greater than 10g of water/100g of particle measured in same manner as described in ASTM Method D281-84 but using water instead of oil. Microsphere refers to spherical particle having average diameter of 0.5 to 50 microns, more preferably from 1 to 15 microns.

The specific surface area of the porous silica is preferably at least 350 m²/g, more preferably from 400 to 1000 m²/g, even more preferably from 550 to 880 m²/g and most preferably from 590 to 810 m²/g.

The porous silica has the capability of absorbing large amounts of oils. Preferably, the porous silica is a porous silica microsphere having an oil absorption value of higher than 100g/100g, more preferably higher than 200g/100g and even more preferably higher than 280g/100g. The oil absorption value refers to the values measured in conformity with ASTM Method D281-84.

The porous silica preferably has an average diameter of 200 nm to 40 microns, more preferably from 0.6 to 25 microns, even more preferably from 1 to 20 microns, still even more preferably from 1.5 to 12 microns and most preferably from 2 to 5 microns. To have a better sensory, the porous silica is preferably substantially uniform in size which means less than 5% of the porous silica have a diameter less than 0.5 times the average diameter and less than 5% of the porous silica have a diameter greater than 1.5 times the average diameter. In another aspect, the range of the diameter of the porous silica is preferably 0.8 to 1.2 times the average diameter, more preferably 0.9 to 1.1 times the average diameter. Particularly preferred porous silica includes MSS-500/3H, MSS-500/H from Kobo Products Inc.

The porous silica is preferably present in amount of 0.01 to 20% by weight of the composition, more preferably from 0.05 to 14%, even more preferably from 0.2 to 9%, still even more preferably from 0.4 to 5% and most preferably from 0.8 to 2% by weight of the composition.

To have a better pore reduction effect and/or optical effect, the weight ratio of polymer particle to the inorganic particle is preferably from 1:1 to 40:1, more preferably from 3:1 to 20:1 and even more preferably from 5:1 to 10:1.

Preferably the composition comprises a thickening agent. A variety of thickening agents may be included in the compositions. Illustrative but not limiting are Acrylamide/Sodium Acryloyldimethyltaurate Copolymer (Aristoflex AVC), Hydroxyethyl Acrylate/Sodium Acryloyldimethyltaurate Copolymer, Aluminum Starch Octenyl Succinate, Polyacrylates (such as Carbomers including Carbopol^{®} 980, Carbopol^{®} 1342, Pemulen TR-2^{®} and the Ultrez^{®} thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Preferably, the thickening agent is selected from Carbomer, taurate copolymer, acrylate copolymer or a mixture thereof. More preferably, the thickening agent is acrylate copolymer.

Amounts of the thickening agent may range, for example, from 0.05 to 10%, more preferably from 0.1 to 5%, even more preferably from 0.3 to 2% by weight of the composition.

The composition may comprise optional ingredients including emollient material, moisturizing agent, organic sunscreen, skin lightening agent, fragrance, natural extract, or a combination thereof.

Particularly preferred moisturizing agents includes, petrolatum, aquaporin manipulating actives, oat kernel flour, substituted urea like hydroxyethyl urea, hyaluronic acid and/or its precursor N-acetyl glucosamine, hyaluronic acid and/or its precursor N-acetyl glucosamine, or a mixture thereof.

A wide variety of organic sunscreen is suitable for use in combination with the essential ingredients of this invention. Suitable UV-A / UV-B sunscreen include, 2- hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof.

Vitamin B3 compounds (including derivatives of vitamin B3) e.g. niacin, nicotinic acid or niacinamide are the preferred skin lightening agent as per the invention, most preferred being niacinamide.

The compositions of the present invention can comprise a wide range of other optional components. Examples include antioxidants, colorants, fragrance, and preservatives.

The composition may comprise water in amount of 10 to 95% by weight of the composition, more preferably from 20 to 88%, even more preferably from 35 to 82%, most preferably from 45 to 75% by weight of the composition.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

Preferably, the personal care composition (product) is a skin care composition (product). The skin care composition (product) refers to a composition (product) suitable for topical application to human skin, preferably is a leave-on product. The term "leave-on" as used with reference to compositions herein means a composition that is applied to or rubbed on the skin, and left thereon. The term "skin" as used herein includes the skin on the face (except eye lids and lips), neck, chest, abdomen, back, arms, under arms, hands, and legs. Preferably "skin" means includes the skin on the face (except eye lids and lips) and under arms, more preferably skin means skin on the face other than lips and eyelids.

The present invention also provides a system to select a personalized skin care composition for the benefit of pore reduction for an individual. The measuring unit may be colorimeter or spectrophotometer. Additionally, or alternatively, the measuring unit may be a mobile terminal incorporating the function of measuring color. In such a way, it is convenient for the consumer to operate.

Preferably, the recommending unit comprises a communication unit, a control unit, and a database. The communication unit may transmit the color information of the individual to the control unit. The control unit may compare the color of the skin surface in need of treatment with a database and identify a skin care composition having substantially same color as the skin surface in need of treatment. The communication unit may transmit the information on the selected skin care composition to the individual. The communication unit may transmit and receive at least one piece of data using a wired/wireless communication. The unit may be module or device.

In a preferred embodiment, the system comprises a mobile terminal, for example a mobile phone, to measure the color and remote server to choose a skin care composition having substantially same color as the skin surface in need of treatment. The mobile terminal and the server may be communication via wireless communication, for example wireless LAN, Wi-Fi or any other suitable way for wireless communication.

The following examples are provided to facilitate an understanding of the invention. The examples are not intended to limit the scope of the claims.

### Examples

### Materials

| Trade name | INCI name | Supplier | Diameter (microns) | Active (wt%) |
|---|---|---|---|---|
| DC9509 | Dimethicone/Vinyldimethicone Crosspolymer (and) CI2-14 Pareth-12 | Dow Corning | 3 | 63 |
| Touch210 | Silica | DSM | 6-15 | 100 |
| CM3K75STC | Titanium Dioxide (And) Cyclopentasiloxane (And) Dimethicone (And) PEG-10 Dimethicone | KOBO | - | 73.5 |
| CM3K65R-LC381 | Iron Oxide (C.I.77491) (and) Cyclopentasiloxane (and) PEG-10 dimethicone (and) Triethoxycaprylylsilane | KOBO | - | 63.25 |
| CM3K65Y-LC182 | Iron Oxide (C.I.77492) (and) Cyclopentasiloxane (and) PEG-10 dimethicone (and) Triethoxycaprylylsilane | KOBO | - | 63 |
| CM3K65EBSI | Iron Oxide (C.I.77499) (and) Cyclopentasiloxane (and) PEG-10 dimethicone (and) Triethoxycaprylylsilane | KOBO | - | 63.5 |
| Simulgel EG | Sodium Acrylate / Sodium Acryloyldimethyl Taurate Copolymer & Isohexadecane & Polysorbate 80 | SEPPIC | - | - |

### Example 1

This example demonstrates the preparation of skin care compositions.

**Table 1**

| Ingredient | Sample (wt% as is in the formulation) | | |
|---|---|---|---|
| | 1 | 2 | 3 |
| Water | To 100 | To 100 | To 100 |
| DC9509 | 37.50 | 37.50 | 37.50 |
| Touch210 | 2.00 | 2.00 | 2.00 |
| CM3K75STC | 2.75 | 2.75 | 2.75 |
| CM3K65R-LC381 | 0.127 | 0.12 | 0.25 |
| CM3K65Y-LC182 | 0.192 | 0.684 | 0.800 |
| CM3K65EBSI | 0.018 | 0.029 | 0.040 |
| Tween 20 | 1.00 | 1.00 | 1.00 |
| Glycerin | 2.00 | 2.00 | 2.00 |
| Simulgel EG | 1.50 | 1.50 | 1.50 |
| Cyclopentasiloxane | 12.00 | 12.00 | 12.00 |
| Phenoxyethanol | 0.50 | 0.50 | 0.50 |
| Disodium EDTA | 0.20 | 0.20 | 0.20 |

A series of skin care compositions having different colors were formulated according to Table 1 by following standard procedure.

### Example 2

This example demonstrated how to choose a suitable skin care composition for a skin with specific color.

The color difference of the skin surface before and after the application of skin care compositions was measured by the following procedure. The L*, a* and b* values of a BioSkin plate (BSP, from Beaulax Co. Ltd., Japan) having a 20# color, were measured by a portable spectrophotometer (CM 2600D, KONICA MINOLTA, Japan) in SCE mode under D65 light source. Then, the samples were evenly spread on the BSP with amount of 2 mg/cm² and dried for 20 minutes at room temperature. Then, the L*, a* and b* values were measured again. The color differences (Delta E) were calculated. The results were included in Table 2.

The performances of pore reduction of each samples were conducted as follows. A commercial BSP of deep pore version (Pore BSP, Code: 10AN, from Beaulax Co. Ltd., Japan) were used as the in vitro substrate mimicking the appearance of human skin. The pore BSP has identical color with the BSP for color different test. The samples were evenly spread with finger cot on pore BSP with dosage of 2 mg/cm². Images were captured under the same settings before the application of samples and after the application of samples for 30 minutes under ambient temperature. Image analysis were conducted based on those images. A parameter of pore CWA (Contrast Weighted Average) was developed to measure and quantify the pore. The contrast for each pixel is the light intensity difference between the pore and the background and calculated by: $C = {L}_{b} - {L}_{f}$ wherein C is the contrast, L_{f} is the light intensity of the pore, and L*_{b}* is the light intensity of the background.

CWA is contrast weighted average of all RGB channels within a specific area. The higher pore CWA, the more visible pores. The pore CWA reduction is calculated by the following equation: $%Pore CWA reduction = \frac{Pore {CWA}_{baseline} - {CWA}_{product}}{Pore {CWA}_{baseline}} X 100 %$

The higher the pore CWA reduction is, the better pore reduction performance is.

The pore reduction performances of all samples are recorded in Table 2.

**Table 2**

| Sample | Delta E | Pore CWA reduction (%) | |
|---|---|---|---|
| | | Average | Standard derivation |
| 1 | 1.63 | 44.1% | 3.7% |
| 2 | 4.37 | 24.4% | 3.9% |
| 3 | 6.06 | 9.8% | 5.6% |

As can be seen from Table 2, it was surprisingly found that the skin care composition having a substantially same color as the skin is capable of providing significant pore reduction benefit. Therefore, a personalized skin care composition for pore reduction may be provided to an individual though selecting a skin care composition which matches the skin color of that individual.

## Claims

1. A method for selecting a personalized skin care composition for providing at least one cosmetic benefit selected from pore reduction and wrinkle reduction, for an individual, the method comprising the steps of:
(i) determining the colour of a skin surface in need of treatment of the individual; and
(ii) choosing a skin care composition having substantially same colour as the skin surface in need of treatment,
wherein a) the composition comprises a polymer particle and b) substantially same colour means that the colour difference (Delta E) of the skin surface before and after the application of the skin care composition with a dosage of 2 mg/cm² is 0 to 5.5 in CIELAB color space.

2. A method for providing at least one cosmetic benefit selected from pore reduction and wrinkle reduction in need of treatment of an individual comprising the step of:
(i) determining the colour of a skin surface in need of treatment of the individual;
(ii) choosing a skin care composition having substantially same colour as the skin surface in need of treatment; and
(iii) applying the skin care composition to the skin surface in need of treatment of the individual,
wherein a) the composition comprises a polymer particle and b) substantially same colour means that the colour difference (Delta E) of the skin surface before and after the application of the skin care composition with a dosage of 2 mg/cm² is 0 to 5.5 in CIELAB color space.

3. The method according to claim 1 or 2 wherein the composition comprises pigment, preferably the pigment comprises coloured pigment and/or white pigment, more preferably the pigment comprises iron oxide and titanium dioxide.

4. The method according to claim 3 the amount of coloured pigment is 0.01 to 2% by weight of the total composition and the white pigment is present in amount of 0.01 to 3% by weight of the composition.

5. The method according to any one of the preceding claims wherein the polymer particle is selected from a silicone elastomer, a polyester or combination thereof, more preferably the polymer particle is selected from dimethicone/vinyl dimethicone crosspolymer, dimethicone crosspolymer and Polysilicone-11, polyester of adipic acid and neopentyl glycol or a mixture thereof.

6. The method according to claim 5 wherein polymer particle polymer particle has an average diameter of from 0.5 to 30 microns.

7. The method according to claim 5 or 6 wherein the polymer particle is present in amount of 5 to 33% by weight of the composition.

8. The method according to any one of the preceding claims wherein the composition comprises inorganic particle, preferably the inorganic particle is a silica, more preferably a porous silica.

9. The method according to claim 8 wherein inorganic particle is present in amount of 0.01 to 20%, preferably from 0.4 to 5% by weight of the composition.

10. The method according to claim 8 or 9 wherein the inorganic particle has an average diameter of 0.6 to 25 microns, more preferably from 1.5 to 12 microns.

11. The method according to any one of the preceding claims wherein the composition comprises 20 to 88% of water by weight of the composition.

12. The method according to any one of the preceding claims wherein step (i) comprises determining all values of L*, a*, and b* of the skin surface in need of treatment.

13. The method according to any one of the preceding claims wherein substantially same colour means that the colour difference (Delta E) of the skin surface before and after the application of the skin care composition with a dosage of 2 mg/cm² is 0 to 4.5, more preferably 0 to 3.4, and even more preferably 0 to 2.5, in CIELAB color space.

14. A system to select a personalized skin care composition for providing at least one cosmetic benefit selected from pore reduction and wrinkle reduction for an individual, comprising:
(i) a measuring unit to determine the colour of skin surface in need of treatment of the individual, and
(ii) a recommending unit to choose a skin care composition having substantially same colour as the skin surface in need of treatment,
wherein a) the composition comprises a polymer particle and b) substantially same colour means that the colour difference (Delta E) of the skin surface before and after the application of the skin care composition with a dosage of 2 mg/cm² is 0 to 5.5 in CIELAB color space.

## Patentansprüche

1. Verfahren zur Auswahl einer personalisierten Hautpflegezusammensetzung zur Erzielung mindestens eines kosmetischen Vorteils, ausgewählt unter Porenverkleinerung und Faltenreduktion, für eine Person, wobei das Verfahren die folgenden Schritte umfasst:
(i) Bestimmung der Farbe einer zu behandelnden Hautoberfläche der Person; und
(ii) Auswählen einer Hautpflegezusammensetzung mit im Wesentlichen derselben Farbe wie die zu behandelnde Hautoberfläche,
wobei a) die Zusammensetzung ein Polymerpartikel umfasst und b) "im Wesentlichen derselben Farbe" bedeutet, dass die Farbdifferenz (Delta E) der Hautoberfläche vor und nach dem Auftragen der Hautpflegezusammensetzung in einer Dosierung von 2 mg/cm² im CIELAB-Farbraum zwischen 0 und 5,5 liegt.

2. Verfahren zur Erzielung mindestens eines kosmetischen Vorteils, ausgewählt unter Porenverkleinerung und Faltenreduktion bei einer zu behandelnden Person, umfassend den Schritt:
(i) Bestimmung der Farbe einer zu behandelnden Hautoberfläche der Person;
(ii) Auswählen einer Hautpflegezusammensetzung mit im Wesentlichen derselben Farbe wie die zu behandelnde Hautoberfläche; und
(iii) Auftragen der Hautpflegezusammensetzung auf die zu behandelnde Hautoberfläche der Person, wobei a) die Zusammensetzung ein Polymerpartikel umfasst und b) "im Wesentlichen derselben Farbe" bedeutet, dass die Farbdifferenz (Delta E) der Hautoberfläche vor und nach dem Auftragen der Hautpflegezusammensetzung in einer Dosierung von 2 mg/cm² im CIELAB-Farbraum zwischen 0 und 5,5 liegt.

3. Verfahren nach Anspruch 1 oder 2, wobei die Zusammensetzung Pigment umfasst, wobei das Pigment vorzugsweise farbiges Pigment und/oder weißes Pigment umfasst, wobei das Pigment bevorzugter Eisenoxid und Titandioxid umfasst.

4. Verfahren nach Anspruch 3, wobei die Menge an farbigem Pigment 0,01 bis 2 Gew.-% der Gesamtzusammensetzung beträgt und das weiße Pigment in einer Menge von 0,01 bis 3 Gew.-% der Zusammensetzung vorliegt.

5. Verfahren nach irgendeinem der vorhergehenden Ansprüche, wobei das Polymerpartikel unter einem Silikonelastomer, einem Polyester oder einer Kombination davon ausgewählt ist, wobei das Polymerpartikel bevorzugter unter Dimethicon/Vinyldimethicon-Crosspolymer, Dimethicon-Crosspolymer und Polysilicon-11, einem Polyester aus Adipinsäure und Neopentylglykol oder einer Mischung davon ausgewählt ist.

6. Verfahren nach Anspruch 5, wobei das Polymerpartikel einen mittleren Durchmesser von 0,5 bis 30 Mikrometern aufweist.

7. Verfahren nach Anspruch 5 oder 6, wobei das Polymerpartikel in einer Menge von 5 bis 33 Gew.-% der Zusammensetzung vorliegt.

8. Verfahren nach irgendeinem der vorhergehenden Ansprüche, wobei die Zusammensetzung ein anorganisches Partikel umfasst, wobei das anorganische Partikel bevorzugt Siliciumdioxid, bevorzugter poröses Siliciumdioxid ist.

9. Verfahren nach Anspruch 8, wobei das anorganische Partikel in einer Menge von 0,01 bis 20 Gew.-%, vorzugsweise von 0,4 bis 5 Gew.-% der Zusammensetzung vorliegt.

10. Verfahren nach Anspruch 8 oder 9, wobei das anorganische Partikel einen mittleren Durchmesser von 0,6 bis 25 Mikrometern, bevorzugter von 1,5 bis 12 Mikrometern, aufweist.

11. Verfahren nach irgendeinem der vorhergehenden Ansprüche, wobei die Zusammensetzung 20 bis 88 Gew.-% Wasser der Zusammensetzung umfasst.

12. Verfahren nach einem der vorhergehenden Ansprüche, wobei Schritt (i) die Bestimmung aller Werte von L*, a* und b* der zu behandelnden Hautoberfläche umfasst.

13. Verfahren nach irgendeinem der vorhergehenden Ansprüche, wobei "im Wesentlichen derselben Farbe" bedeutet, dass die Farbdifferenz (Delta E) der Hautoberfläche vor und nach dem Auftragen der Hautpflegezusammensetzung in einer Dosierung von 2 mg/cm² im CIELAB-Farbraum zwischen 0 und 4,5, bevorzugter zwischen 0 und 3,4 und noch bevorzugter zwischen 0 und 2,5 liegt.

14. System zur Auswahl einer personalisierten Hautpflegezusammensetzung zur Erzielung mindestens eines kosmetischen Vorteils, ausgewählt unter Porenverkleinerung und Faltenreduktion, für eine Person, umfassend:
(i) eine Messeinheit zur Bestimmung der Farbe der zu behandelnden Hautoberfläche der Person und
(ii) eine Empfehlungseinheit zur Auswahl einer Hautpflegezusammensetzung mit im Wesentlichen derselben Farbe wie die zu behandelnde Hautoberfläche,
wobei a) die Zusammensetzung ein Polymerpartikel umfasst und b) "im Wesentlichen derselben Farbe" bedeutet, dass die Farbdifferenz (Delta E) der Hautoberfläche vor und nach dem Auftragen der Hautpflegezusammensetzung in einer Dosierung von 2 mg/cm² im CIELAB-Farbraum zwischen 0 und 5,5 liegt.

## Revendications

1. Procédé pour sélectionner une composition de soin de la peau personnalisée pour apporter au moins un bénéfice cosmétique choisi parmi la réduction des pores et la réduction des rides, pour un individu, le procédé comprenant les étapes de :
(i) détermination de la couleur d'une surface de peau de l'individu nécessitant un traitement ; et
(ii) choix d'une composition de soin de la peau ayant pratiquement la même couleur que la surface de peau nécessitant un traitement,
dans lequel a) la composition comprend une particule polymère et b) par pratiquement la même couleur, on entend que la différence de couleur (Delta E) de la surface de peau avant et après l'application de la composition de soin de la peau à une dose de 2 mg/cm² est de 0 à 5,5 dans l'espace colorimétrique CIELAB.

2. Procédé pour apporter au moins un bénéfice cosmétique choisi parmi la réduction des pores et la réduction des rides nécessitant un traitement chez un individu, comprenant les étapes de :
(i) détermination de la couleur d'une surface de peau de l'individu nécessitant un traitement ;
(ii) sélection d'une composition de soin de la peau ayant pratiquement la même couleur que la surface de peau nécessitant un traitement ; et
(iii) application de la composition de soin de la peau sur la surface de peau de l'individu nécessitant un traitement,
dans lequel a) la composition comprend une particule polymère et b) par pratiquement la même couleur, on entend que la différence de couleur (Delta E) de la surface de peau avant et après l'application de la composition de soin de la peau à une dose de 2 mg/cm² est de 0 à 5,5 dans l'espace colorimétrique CIELAB.

3. Procédé selon la revendication 1 ou 2, dans lequel la composition comprend un pigment, de préférence le pigment comprend un pigment coloré et/ou un pigment blanc, mieux encore le pigment comprend de l'oxyde de fer et du dioxyde de titane.

4. Procédé selon la revendication 3, dans lequel la quantité de pigment coloré est de 0,01 à 2 % en poids de la composition totale et le pigment blanc est présent en une quantité de 0,01 à 3 % en poids de la composition.

5. Procédé selon l'une quelconque des revendications précédentes, dans lequel la particule de polymère est choisie parmi un élastomère de silicone, un polyester et une combinaison de ceux-ci, mieux encore la particule de polymère est choisie parmi un polymère réticulé de diméthicone/vinyldiméthicone, un polymère réticulé de diméthicone et de Polysilicone-11, un polyester d'acide adipique et de néopentylglycol, et leurs mélanges.

6. Procédé selon la revendication 5, dans lequel la particule de polymère a un diamètre moyen de 0,5 à 30 micromètres.

7. Procédé selon la revendication 5 ou 6, dans lequel la particule de polymère est présente en une quantité de 5 à 33 % en poids de la composition.

8. Procédé selon l'une quelconque des revendications précédentes, dans lequel la composition comprend une particule inorganique, de préférence la particule inorganique est une silice, mieux encore une silice poreuse.

9. Procédé selon la revendication 8, dans lequel la particule inorganique est présente en une quantité de 0,01 à 20 %, de préférence de 0,4 à 5 % en poids de la composition.

10. Procédé selon la revendication 8 ou 9, dans lequel la particule inorganique a un diamètre moyen de 0,6 à 25 micromètres, mieux encore de 1,5 à 12 micromètres.

11. Procédé selon l'une quelconque des revendications précédentes, dans lequel la composition comprend 20 à 88 %, en poids de la composition, d'eau.

12. Procédé selon l'une quelconque des revendications précédentes, dans lequel l'étape (i) comprend la détermination de toutes les valeurs de L*, a* et b* de la surface de la peau ayant besoin d'un traitement.

13. Procédé selon l'une quelconque des revendications précédentes, dans lequel, par pratiquement la même couleur, on entend que la différence de couleur (Delta E) de la surface de peau avant et après l'application de la composition de soin de la peau à une dose de 2 mg/cm² est de 0 à 4,5, mieux encore de 0 à 3,4 et plus particulièrement de 0 à 2,5 dans l'espace colorimétrique CIELAB.

14. Système pour sélectionner une composition de soin de la peau personnalisée pour apporter au moins un bénéfice cosmétique choisi parmi la réduction des pores et la réduction des rides, pour un individu, comprenant :
(i) une unité de mesure pour déterminer la couleur d'une surface de peau de l'individu nécessitant un traitement, et
(ii) une unité de recommandation pour choisir une composition de soin de la peau ayant pratiquement la même couleur que la surface de peau nécessitant un traitement,
dans lequel a) la composition comprend une particule polymère et b) par pratiquement la même couleur, on entend que la différence de couleur (Delta E) de la surface de peau avant et après l'application de la composition de soin de la peau à une dose de 2 mg/cm² est de 0 à 5,5 dans l'espace colorimétrique CIELAB.
